# EUROPEAN PATENT APPLICATION

(11) **EP 4 492 392 A1**
(43) Date of publication of application: **15.01.2025**
(21) Application number: 23779340.1
(22) Date of filing: 08.03.2023
(51) Int. Cl.: G16H 20/00, G16H 50/20

(54) **PROGRAM, INFORMATION PROCESSING DEVICE, AND INFORMATION PROCESSING METHOD**

(30) Priority: 28.03.2022 JP 2022052272
(71) Applicant: Terumo Kabushiki Kaisha, Tokyo 151-0072 (JP)
(72) Inventor: NISHIMURA, Toshihiko, Ashigarakami-gun, Kanagawa 259-0151 (JP); HONMA, Yasuyuki, Ashigarakami-gun, Kanagawa 259-0151 (JP)
(74) Representative: Prüfer & Partner mbB Patentanwälte · Rechtsanwälte
(86) International application number: PCT/JP2023/008731
(87) International publication number: WO 2023/189313

(57) **Abstract**

A program or the like is provided that switches a method for acquiring biological information, in a stepwise manner, according to an abnormality degree of a health state. The program causes a computer to execute processing for determining an abnormality of a subject on a basis of first sensor information obtained from a first sensor and determining the abnormality of the subject on a basis of second sensor information obtained from a second sensor having a higher physical privacy disclosure degree than the first sensor or executing a test program regarding the abnormality on the subject, according to a determination result.

## Description

### Technical Field

The present invention relates to a program, an information processing device, and an information processing method.

### Background Art

Several techniques for acquiring biological information such as a facial expression, an utterance, or a pulse and determining an abnormality in a health state have been proposed. For example, there is a health support system that installs sensors in a house or a nursing care facility where a subject who needs health observation stays and acquires biological information of the subject over time (Patent Literature 1).

### Citation List

### Patent Literature

Patent Literature 1: JP 2018-094242 A

### Summary of Invention

### Technical Problem

However, according to the invention disclosed in Patent Literature 1, it is necessary to constantly monitor the subject using a camera, a microphone, or the like, and there is a problem from the viewpoint of ensuring privacy. On the other hand, if ensuring the privacy is emphasized, biological information sufficient for grasping a health state is not acquired. From such a current situation, it is difficult to balance between health observation and ensuring privacy.

In one aspect, an object is to provide a program or the like that switches a method for acquiring biological information according to an abnormality degree of a health state in a stepwise manner.

### Solution to Problem

A program according to one aspect determines an abnormality of a subject on a basis of first sensor information obtained from a first sensor and determines the abnormality of the subject on a basis of second sensor information obtained from a second sensor having a higher physical privacy disclosure degree than the first sensor or executes a test program regarding the abnormality on the subject, according to a determination result.

### Advantageous Effects of Invention

In one aspect, it is possible to provide a program or the like that switches a method for acquiring biological information according to an abnormality degree of a health state in a stepwise manner.

### Brief Description of Drawings

Fig. 1 is a schematic diagram illustrating an outline of a health abnormality determination system.
Fig. 2 is a schematic diagram illustrating a configuration example of an information processing device.
Fig. 3 is an explanatory diagram illustrating a data layout of a health state DB.
Fig. 4 is a flowchart illustrating a processing procedure of a program executed by the information processing device.
Fig. 5 is a flowchart illustrating the processing procedure of the program executed by the information processing device.
Fig. 6 is a flowchart illustrating the processing procedure of the program executed by the information processing device.
Fig. 7 is a flowchart illustrating the processing procedure of the program executed by the information processing device.
Fig. 8 is a flowchart illustrating the processing procedure of the program executed by the information processing device.
Fig. 9 is a flowchart illustrating the processing procedure of the program executed by the information processing device.
Fig. 10 is a flowchart illustrating the processing procedure of the program executed by the information processing device.
Fig. 11 is a flowchart illustrating the processing procedure of the program executed by the information processing device.
Fig. 12 is a flowchart illustrating the processing procedure of the program executed by the information processing device.
Fig. 13 is a flowchart illustrating an example of a processing procedure of a program executed by an information processing device according to a sixth embodiment.

### Description of Embodiments

In the present embodiment, a health abnormality determination system that achieves both of ensuring privacy and a function for determining an abnormality of a health state will be described with reference to the drawings from a first to fifth embodiments.

### [First Embodiment]

In a first embodiment, a mode will be described in which sensors are switched in a stepwise manner to detect pieces of biological information having different privacy disclosure degrees.

Here, description is made as assuming that the privacy disclosure degree is a level indicating how much information that the subject does not want to let others know is disclosed to the others.

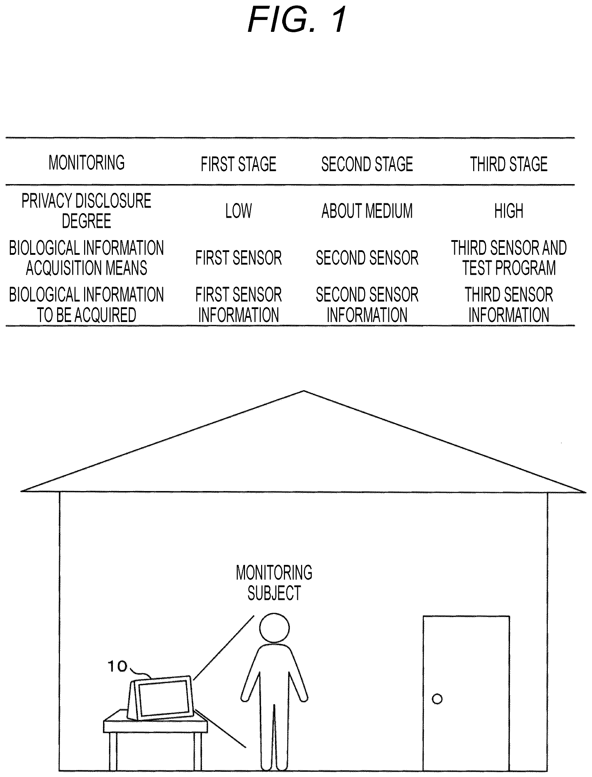

Fig. 1 is a schematic diagram illustrating an outline of a health abnormality determination system.

In this system, an information processing device 10 is installed in a living space of a subject who needs health observation. As the living space of the subject, for example, a house, a nursing care facility, a medical facility, or the like is exemplified. The subject provides the biological information to the information processing device 10 so that a health state of the subject is monitored while the subject is living a normal life.

The information processing device 10 is dedicated hardware including a sensor that acquires the biological information. The information processing device 10 may be a general-purpose information device used by the subject such as a smartphone, a tablet terminal, or a smart speaker.

The biological information acquired by the information processing device 10 includes biological information with a high physical privacy disclosure degree and biological information with a low physical privacy disclosure degree.

The former biological information is obtained by detailed monitoring of private life, and a privacy protection degree is low. When the biological information is known by the others, there is a possibility that a specific individual is identified. Examples of the biological information include personal information such as information regarding a health state associated with an individual, a face image, or a stress check. When the biological information is used for the health observation, accuracy of abnormality determination on the health state is improved. However, there is a limit on ensuring the privacy of the person.

On the other hand, the latter biological information is obtained by simple monitoring of the private life, and the privacy protection degree is high. Even if the biological information is known by the others, it is difficult to identify a specific individual. Examples of the biological information include personal information such as daily life sound, an image other than the face, a body shape, or a skeleton. When the biological information is used for the health observation, the privacy of the person is ensured. However, the accuracy of the abnormality determination of the health state is limited.

Based on such a background, the information processing device 10 used in this system executes processing for switching the sensor, in a stepwise manner, used to acquire the biological information.

As a first stage, the information processing device 10 acquires the biological information with the low physical privacy disclosure degree by a first sensor. Using the biological information acquired at the first stage as a trigger, the information processing device 10 sequentially switches to acquisition of the biological information with the high physical privacy disclosure degree. If an abnormality is not found in the first stage, the first stage is not switched to a second stage or subsequent stages, the privacy of the subject is ensured. Furthermore, depending on a degree of the abnormality detected at the first stage, the second stage may be skipped, and switching to a third stage may be performed. That is, by switching the first sensor to the third sensor in a stepwise manner, it is possible to achieve both of ensuring the privacy and a function for determining the abnormality in the health state. Note that, in order to achieve both of ensuring the privacy and the abnormality determination of the health state, by providing a difference in a recording or output stage for the biological information using the first sensor to the third sensor, necessary determination for improving accuracy regarding the health state may be performed, while avoiding unnecessary privacy disclosure.

In the present embodiment, description will be made as assuming that the information processing device 10 performs three-stage switching. However, two stages or four or more stages of switching may be performed.

Note that, in the present embodiment, description will be made as assuming that the single information processing device 10 configures the present system. However, the present system may be implemented by a plurality of pieces of hardware installed in the living space in cooperation with each other. Furthermore, a part of the processing (for example, health abnormality determination processing) executed by the information processing device 10 may be executed by a server on a cloud.

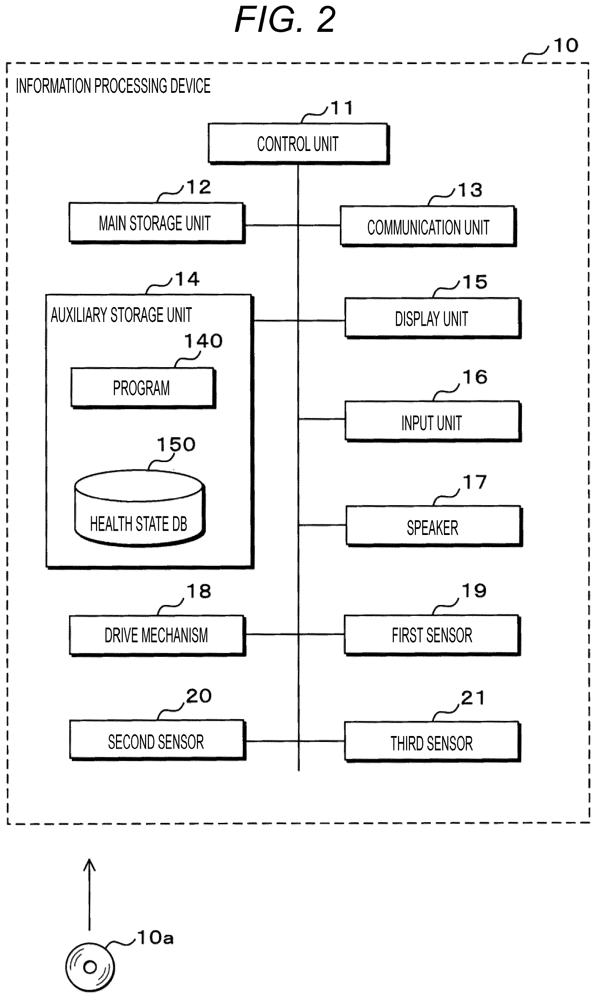

Fig. 2 is a block diagram illustrating a configuration example of the information processing device 10.

The information processing device 10 includes a control unit 11, a main storage unit 12, a communication unit 13, an auxiliary storage unit 14, a display unit 15, an input unit 16, a speaker 17, a drive mechanism 18, a first sensor 19, a second sensor 20, and a third sensor 21.

The control unit 11 is one or two or more processors such as central processing units (CPU), micro-processing units (MPU), graphics processing units (GPU), or quantum processors and executes various types of information processing.

The main storage unit 12 is a temporary storage region such as a static random access memory (SRAM) or a dynamic random access memory (DRAM) and temporarily stores data necessary for the control unit 11 to execute processing.

The communication unit 13 is a communication interface to connect to a communication network such as the Internet or a local area network (LAN).

The auxiliary storage unit 14 is a memory such as a solid state drive (SSD) or a hard disk drive (HDD). The auxiliary storage unit 14 stores a program 140 (program product) for causing the information processing device 10 to execute processing, a health state data base (DB) 150, a plurality of test programs, and other pieces of data.

The auxiliary storage unit 14 stores biological information of the subject when the subject is healthy. Specifically, the biological information includes a face image, a height, a weight, a blood pressure, a heart rate, a walking motion picture, or the like of the subject. The control unit 11 determines a health abnormality of the subject, on the basis of the biological information stored in the auxiliary storage unit 14. The auxiliary storage unit 14 may store a statistic amount or a feature amount based on the biological information, in addition to the specific biological information.

Moreover, the auxiliary storage unit 14 may store a machine learning model constructed on the basis of the statistic amount or the feature amount based on the biological information, instead of storing the biological information when the subject is healthy. If the machine learning model is stored, it is not necessary to store the specific biological information or the statistic amount or the feature amount based on the biological information. Specifically, in a case where sensor information is input, it is sufficient to store a machine learning model such as a support vector machine (SVM), a decision tree, a neural network, or a long short term memory (LSTM) that is learned so as to output a classification result indicating whether or not the information is normal.

Note that the information processing device 10 includes a reading unit that reads a portable storage medium 10a and may read the program 140 from the portable storage medium 10a. Furthermore, the information processing device 10 may download the program 140 from another computer via a communication network.

The display unit 15 is a display screen such as a liquid crystal display or an organic electro luminescence (EL) display. The display unit 15 displays an image of a screen on which a test program regarding an abnormality to be executed on the subject to be described later is executed or a screen indicating a result of determining the abnormality of the subject.

The input unit 16 is an input interface such as a touch panel or a mechanical operation button. The input unit 16 receives an operation input from the subject. Specifically, the input unit 16 receives the biological information when the subject is healthy or the like. The input unit 16 may be a microphone that collects a voice command of the subject.

The speaker 17 is a device that outputs a voice such as a voice message or an alert. The speaker 17 may output a voice instruction to the subject, when the test program is executed.

The drive mechanism 18 rotationally drives a main body of the information processing device 10 in horizontal and vertical directions. The drive mechanism 18 is controlled by the control unit 11, in accordance with a level of the privacy disclosure degree when the subject is monitored.

The control unit 11 switches each sensor according to the level of the privacy disclosure degree. The first sensor 19, the second sensor 20, and the third sensor 21 are set in ascending order of the privacy disclosure degree.

In the present embodiment, description will be made as assuming that each sensor is built in the information processing device 10. However, each sensor may be an external sensor. Furthermore, first sensor information may be acquired by the subject wearing a wearable terminal.

The first sensor 19 acquires the first sensor information including biological information with a low privacy disclosure degree. The information processing device 10 includes a plurality of types of sensors, for example, a microphone, a light detection and ranging (LiDAR), an event drive imaging device, a camera, or the like. The control unit 11 acquires target biological information by switching ON/OFF of the plurality of first sensors 19.

In a case where the control unit 11 activates the camera as the first sensor 19, the camera functions as a wide-angle camera. At this time, the first sensor 19 is controlled by the drive mechanism 18 and does not image a face of the subject or does not zoom in the face of the subject. This is to keep the privacy disclosure degree low. In a case where the control unit 11 activates the camera as the first sensor 19, conversely, the control unit 11 may perform to control such that only the face of the subject is captured in an imaging range (region of interest). This is because it can be considered that the privacy disclosure degree can be lowered by making action content of the subject be unidentifiable by imaging only the face.

For the first sensor 19, a so-called intelligent vision sensor, which is a camera unit in which an AI function for executing processing for recognizing an object and a person is built-in, may be used. Although this camera unit images the face of the subject, the camera unit does not output the imaged image. This camera unit only outputs tracking data of the subject in the imaging range, an eye line by tracking, a line connecting corners of the mouth, position coordinates in an image of a feature point (positions of eyes, nose, and mouth) of the face of the subject, angle information, and a recognition result (person or the like). Moreover, this camera unit may also perform abnormality determination related to health to be described later.

The second sensor 20 acquires second sensor information including biological information with a medium privacy disclosure degree. In a case where the second sensor 20 is a camera, the second sensor 20 functions as an imaging device that images the face of the subject at a narrower angle than the first sensor 19. This is to make the privacy disclosure degree be higher than the first stage and to acquire the face image as the biological information.

The third sensor 21 acquires third sensor information including biological information with a high privacy disclosure degree. The third sensor 21 is a combination of a camera and a microphone for acquiring biological information for the test program. The camera used for the third sensor 21 has a narrower angle than the camera used for the second sensor 20 and acquires a zoomed face image, arm image, or the like of the subject.

In addition, the biological information may be acquired by activating a LiDAR as the first sensor 19, a wide-angle camera as the second sensor 20, and a narrow-angle camera as the third sensor 21.

The test program is diagnostic processing to acquire biological information with a higher privacy disclosure degree than the first sensor information and the second sensor information. The test program is, for example, a medical interview, motion analysis, or a stroke (cerebrovascular disorder) diagnosis (for example, Cincinnati Prehospital Stroke Scale (CPSS), National Institutes of Health Stroke Scale (NIHSS), or Kurashiki Prehospital Scale (KPSS)), or the like.

The medical interview includes heart failure self-check, epilepsy check, stress check, or the like. The information processing device 10 determines a health abnormality of the subject according to a result of each medical interview.

The motion analysis includes, for example, walking analysis. The walking analysis is an analysis result based on biological information regarding walking, such as symmetry of walking, movement of the center of gravity, a motion of a joint, or how to use a cane. The information processing device 10 determines a motor function of the subject according to the result of the walking analysis.

The diagnosis of neurological disorders such as stroke includes facial distortion determination, a diagnosis of upper limb elevation, and determination of neurological symptoms such as dysarthria. The information processing device 10 outputs a possibility of the stroke of the subject or severity of the neurological symptom as a probability or a score, according to the diagnosis result. Details of the stroke diagnosis will be described later in a fifth embodiment.

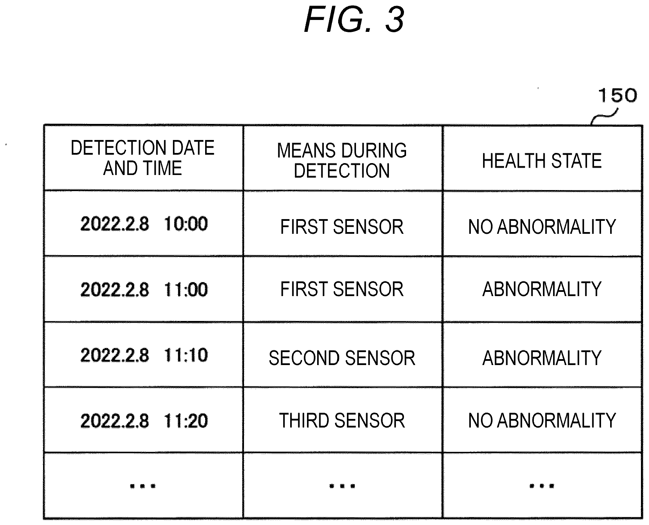

Fig. 3 is an explanatory diagram illustrating a data layout of the health state DB 150. The health state DB 150 is a DB that stores fields of a detection date and time, means during detection, and the health state. The health state DB 150 stores a health state of a monitoring target in time series.

In the detection date and time field, a date and time when the biological information of the subject is detected is stored. In the means during detection field, a type of a sensor that monitors the subject is stored. In the health state field, a health state of the subject such as "abnormal" or "not abnormal" is stored.

The information processing device 10 compares the first sensor information or the second sensor information indicating the abnormality of the subject with biological information at the healthy time stored in the auxiliary storage unit 14 and detects a biological information abnormality, facial palsy, a mental abnormality, falling, shock or thrill, faintness, or an utterance abnormality. Specific symptoms of the biological information abnormality include an abnormality in pulse, heart rate variability, respiration, blood oxygen level, or blood pressure variability.

Hereinafter, the biological information acquired by each sensor and the method for determining the health abnormality performed by the information processing device 10 are exemplified. As the sensor that acquires the biological information, for example, a LiDAR, a camera, a microphone, an event drive imaging device, a millimeter wave sensor, an ultrasonic sensor, a thermographic camera, or the like is exemplified.

### [LiDAR]

After irradiating an object with infrared rays, the LiDAR calculates a distance to the object on the basis of a round-trip time from when being reflected to when the infrared rays return to the own device. The LiDAR outputs point cloud data as three-dimensional information of the object. For example, in a case where the LiDAR irradiates the face of the subject with the infrared rays, it is possible to acquire a three-dimensional image including unevenness information of the face.

The information processing device 10 determines the facial palsy, on the basis of the three-dimensional image of the face acquired by the LiDAR. Specifically, the unevenness information of the face of the subject is compared with an unevenness state at the healthy time. In a case where the unevenness information of the face of the subject is different from the unevenness state at the healthy time by a predetermined threshold or more, it is determined that the subject has facial palsy. On the other hand, in a case where the unevenness information is within a range of the unevenness state at the healthy time, it is determined that the subject does not have the facial palsy.

### [Camera]

The camera acquires a face image, an arm image, or a leg image. The information processing device 10 determines the facial palsy, the biological information abnormality, the mental abnormality, the faintness, or the falling, on the basis of the biological information acquired by the camera.

The facial palsy indicates symptoms such as the face looks bilaterally asymmetric, the forehead cannot be wrinkled, the eyebrows cannot be raised, the eyelids cannot be closed, or the corners of the mouth cannot be raised. This is because a healthy portion where expression muscles can be moved and paralytic (abnormal) portion where the expression muscles cannot be moved are generated in the face.

For example, in a case where the left side of the face is healthy and the right side of the face is paralyzed, even if the subject tries to smile, the subject cannot make a smile with the corner of the mouth on the right side raised, and normal smile expression cannot be made.

By performing a known Yanagihara grading system or the like used for an examination of the facial palsy, the information processing device 10 determines the facial palsy on the basis of a contour or wrinkle information of the face acquired by the camera. Specifically, a score according to a curvature of the contour, the number or a depth of the wrinkles on the forehead, or a change amount of a depth or a length of a nasolabial line is calculated so as to determine the facial palsy. In a case where the intelligent vision sensor is used as the first sensor 19, the information processing device 10 may calculate a score on the basis of information output from the intelligent vision sensor, without deriving the information such as the feature point by the control unit 11.

The subject changes the facial expression according to an instruction of the Yanagihara grading system. Instruction content is guided by voice from the speaker 17 or a text from the display unit 15.

The information processing device 10 extracts a part (forehead, eyebrow, eye, cheek, corners of the mouth, or the like) of the face, as the feature point, from the face image. When the expression of the face is changed, for example, the feature amount such as the curvature of the contour, the number or the depth of the wrinkles on the forehead, or the depth or the length of the nasolabial line changes. The information processing device 10 specifies a change amount of the feature amount at the feature point that changes before and after the change of the expression. The information processing device 10 sets bilateral symmetry as a score according to the specified change amount and determines the facial palsy.

Note that the determination on the facial palsy may be performed using a machine learning model such as a neural network, an SVM, a transformer, or an LSTM. In this case, the control unit 11 specifies presence/absence of the facial palsy or a level of the facial palsy by inputting the face image of the subject in a model that is learned to classify the face image into any one of "normal", "facial palsy level is low", or "facial palsy level is high" when the face image is input.

The information processing device 10 determines the mental abnormality or fatigue from the face image (for example, skin color) acquired by the camera.

The skin color changes from moment to moment according to a blood flow. Since hemoglobin included in the blood has a property for absorbing visible light, an RGB signal in a face region periodically changes.

The information processing device 10 detects a change in the skin color from the face image acquired by the camera. In a case where a frequency is different from a frequency at the healthy time by a predetermined threshold or more on the basis of time-series data regarding the change in the skin color, the information processing device 10 determines that autonomic nerve is disturbed. The information processing device 10 determines the mental abnormality or the fatigue felt by the subject, according to a disturbance degree of the autonomic nerve.

Note that, when the mental abnormality or the fatigue felt by the subject is determined, the machine learning model such as the neural network, the SVM, the transformer, or the LSTM may be used. In this case, the control unit 11 determines the mental abnormality or the fatigue felt by the subject, by inputting the face image of the subject acquired by the camera into the model that is learned to classify the face image into any one of "mental abnormality", "fatigue", or "healthy" when the face image is input.

Faintness of a body indicates a state where no force is applied to hands or feet and causes a symptom such as it is hard to handle chopsticks as intended or it is hard to walk. For example, in a case where a state where an object held by the subject is not held any more by separating the object from the hand or the like is detected in the arm image acquired from the camera, the information processing device 10 determines that an abnormality of the faintness occurs.

### [Microphone]

The microphone acquires voice information of the subject. The information processing device 10 determines falling or an utterance abnormality on the basis of the voice information acquired by the microphone.

In a case where a daily life sound acquired by the microphone is equal to or more than the predetermined threshold, the information processing device 10 determines that the subject has fallen. For example, in a case where a volume (for example, sound of moving furniture or sound of walking on the floor) equal to or less than the predetermined threshold is acquired, the information processing device 10 determines that "not falling". On the other hand, in a case where a volume (for example, sound of falling on the floor or sound of falling down the stairs) equal to or more than the predetermined threshold is acquired, it is determined that "falling has occurred".

The information processing device 10 executes an utterance test and determines the utterance abnormality by determining whether or not a predetermined word can be uttered without delay. The predetermined word is, for example, "pataka" or "ruri and haruri both shine when being lighted". The utterance abnormality is determined on the basis of a voice spoken by the subject toward the microphone.

Note that the information processing device 10 may determine the utterance abnormality using an utterance (for example, daily uttered voice) of the subject acquired from the microphone, instead of executing the utterance test. In this case, the information processing device 10 compares a feature amount extracted from the utterance of the subject and a feature amount based on an utterance pattern at the healthy time stored in the auxiliary storage unit 14. In a case where the feature amount extracted from the utterance of the subject is different from the feature amount at the healthy time, the information processing device 10 determines that the utterance abnormality occurs.

For the determination on the utterance abnormality, for example, an utterance clarity that is an index used to evaluate an utterance function is used. The utterance clarity is graded as "clear", "sometimes there are words that cannot be heard", "sometimes there are words that can be heard", "unclear", or the like according to content uttered by the subject. The information processing device 10 specifies the utterance clarity of the subject by voice recognition. In a case of specifying that the utterance content of the subject is clear, the information processing device 10 determines that there is no utterance abnormality. On the other hand, regarding the utterance content of the subject, in a case where it is specified that sometimes there are words that cannot be heard, sometimes there are words that can be heard, or unclear, it is determined that the utterance abnormality occurs.

Note that the determination on the utterance abnormality of the subject may be performed using the machine learning model such as the neural network, the SVM, the transformer, or the LSTM. In this case, the control unit 11 determines the utterance abnormality of the subject by inputting the voice information of the subject acquired by the microphone, into the model learned to classify the voice information into "utterance abnormality" or "no utterance abnormality" when the voice information is input.

### [Event drive imaging device]

After extracting a luminance change (for example, movement of subject) of the subject, the event drive imaging device outputs only a pixel where the luminance change occurs in combination with position information and time information. The event drive imaging device does not extract a pixel (for example, background portion) where the luminance change does not occur.

The shock is a symptom caused by dizziness or the like, and the thrill is a symptom caused by Parkinson's disease or the like. A frequency of the thrill is higher than that of the shock. In a case where the subject has the shock such as body wobbling or the thrill such as hand shaking, the event drive imaging device detects these movements.

Specifically, the event drive imaging device extracts only a portion where the shock or the thrill of the subject occurs and detects a frequency distribution. In a case where a frequency of each pixel acquired by the event drive imaging device is equal to or more than the predetermined threshold or in a case where asymmetry property occurs in the frequency of each pixel acquired by the event drive imaging device, the information processing device 10 determines that the shock or the thrill occurs. On the other hand, in a case where the frequency is not equal to or more than the predetermined threshold, it is determined that the shock or the thrill does not occur.

Note that whether or not the shock or the thrill occurs may be determined using the machine learning model such as the neural network, the SVM, the transformer, or the LSTM. In this case, the control unit 11 determines whether or not the shock or the thrill occurs by inputting the luminance change of the subject acquired by the event drive imaging device, into a model that has learned to classify the luminance change into "shock or thrill occurs" or "no shock or thrill" when the luminance change of the subject is input.

### [Millimeter wave sensor]

The millimeter wave sensor detects a position and a speed of the moving object. The information processing device 10 acquires a head position of the subject and a speed when the head position moves. In a case where the head position changes by a value equal to or more than the predetermined threshold within a certain period of time, the information processing device 10 determines that the subject has fallen.

### [Ultrasonic sensor]

The ultrasonic sensor detects a distance from the own device to the object and whether or not the object exists. The information processing device 10 acquires a distance from the own device to the head position of the subject and whether or not the head position exists. In a case where the head position changes from a predetermined range within a certain period of time, the information processing device 10 determines that the subject has fallen.

### [Thermographic camera]

The thermographic camera converts radiant energy from the object into a temperature and measures a temperature distribution on an object surface. The information processing device 10 detects a region where a temperature acquired in the imaging range is equal to or higher than a threshold as a region where the monitoring target exists. The information processing device 10 specifies that the subject is in a standing position or a sitting position on the basis of a shape of the detected region. In a case where the specified standing or sitting position changes by a value equal to or more than the predetermined threshold within a certain period of time, the information processing device 10 determines that the subject has fallen.

Note that falling of the subject may be determined using the machine learning model such as a convolutional neural network (CNN), the SVM, the transformer, or the LSTM. In this case, the control unit 11 determines the falling of the subject by inputting data acquired by the millimeter wave sensor, the ultrasonic sensor, or the thermographic camera into a model that is learned to classify the data into "falling" or "not falling" when the data acquired by the millimeter wave sensor, the ultrasonic sensor, and the thermographic camera is input.

### [Camera, LiDAR, millimeter wave sensor, and ultrasonic sensor]

When acquiring biological information regarding the respiration or a heartbeat of the subject, the information processing device 10 activates the camera, the LiDAR, the millimeter wave sensor, or the ultrasonic sensor. The information processing device 10 analyzes a shape of a displacement portion from a value detected by the camera, the LiDAR, the millimeter wave sensor, or the ultrasonic sensor and calculates a heartbeat, a heart rate variability, a magnitude of pulse shake, a peak time of the heartbeat, or the like in the displacement portion.

The information processing device 10 determines whether or not there is an abnormality in the heart or the blood vessel, by determining whether or not each calculated numerical value is equal to or more than the predetermined threshold. As the abnormality in the heart or the blood vessel, for example, brain infarction, brain hemorrhage, heart failure, atrial fibrillation, foot infarction, varicose vein, blood clot, or the like is exemplified. In this case, the pulse or the heart rate variability may be compared with data acquired by the wearable terminal.

### [Camera and microphone]

The information processing device 10 activates the camera and the microphone when determining the facial palsy. The information processing device 10 determines the facial palsy by combining the camera and the microphone. Details of the facial palsy determination will be described later in the fifth embodiment.

As described above, the information processing device 10 determines the health abnormality of the subject being monitored, on the basis of the biological information acquired by each sensor. As the health abnormality, for example, a health abnormality as symptoms of the stroke is exemplified.

Next, processing for switching each sensor in a stepwise manner will be described. Here, the first stage, the second stage, and the third stage are set in ascending order of the privacy disclosure degree of the biological information to be acquired.

### [Health abnormality determination at first stage]

The control unit 11 activates the first sensor 19 and detects the first sensor information with a low physical privacy disclosure degree. The first sensor 19 is, for example, the microphone, the LiDAR, the event drive imaging device, the camera, or the like. The first sensor 19 may be a so-called intelligent vision sensor. The control unit 11 stores a type of the activated first sensor 19 and the number of activated first sensors 19 in the auxiliary storage unit 14.

The sensor such as the LiDAR, the event drive device, or the camera functions as an imaging device that does not image the face of the subject or has a wide angle to such an extent that the face cannot be identified. Therefore, the privacy disclosure degree of the acquired biological information is low. Note that, as described above, even in a case where only the face of the subject is set as the imaging range conversely and the action content cannot be identified, the first sensor 19 has a low privacy disclosure degree.

The plurality of types of first sensors 19 are included in the information processing device 10. The control unit 11 acquires target first sensor information from the first sensor 19. The control unit 11 determines whether or not there is an abnormality in the health of the subject on the basis of the first sensor information.

In a case of determining that there is an abnormality in the health of the subject, the control unit 11 switches detection means from the first sensor 19 to the second sensor 20, because more detailed monitoring is needed. When the detection means is switched, the control unit 11 may cause the display unit 15 to display the switching of the sensor. This is to consider the privacy of the subject.

In a case of determining that there is no abnormality in the health of the subject, the control unit 11 continues to acquire the biological information by the first sensor 19. This is to ensure the privacy of the subject.

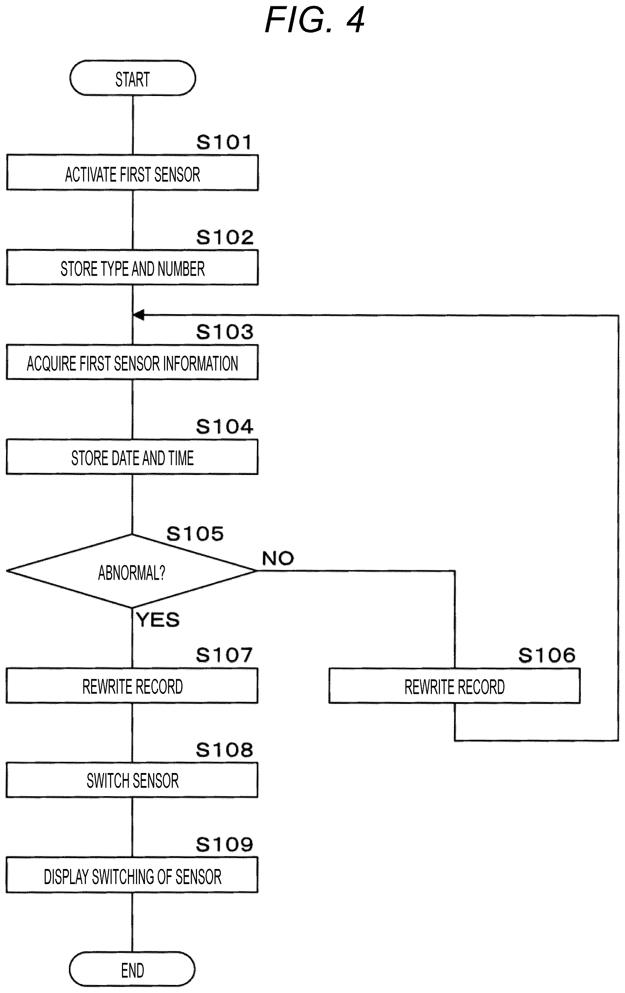

Fig. 4 is a flowchart illustrating a processing procedure of the program 140 executed by the information processing device 10. The control unit 11 of the information processing device 10 executes the following processing on the basis of the program 140.

The control unit 11 activates the first sensor 19 (step S101). The control unit 11 stores the type and the number of activated first sensors 19 (step S102). The first sensor 19 detects the first sensor information of the subject. The control unit 11 acquires the first sensor information detected by the first sensor 19 (step S103). The control unit 11 stores a date and time when the first sensor information is detected in the health state DB 150 (step S104).

The control unit 11 determines an abnormality in the health state according to each method described above, on the basis of the acquired first sensor information (step S105). In a case of determining that there is no abnormality in the health state (step S105: NO), the control unit 11 rewrites a record in the health state field to "no abnormality" (step S106). The control unit 11 returns to the processing in step S103.

In a case of determining that there is an abnormality in the health state (step S105: YES), the control unit 11 rewrites the record in the health state field to "abnormal" (step S107). The control unit 11 switches the detection means from the first sensor 19 to the second sensor 20 (step S108). The control unit 11 makes the display unit 15 display the activation of the second sensor 20 (step S109).

### [Health abnormality determination at second stage]

The control unit 11 proceeds to abnormality determination processing by the second sensor 20 with a higher physical privacy disclosure degree than the first sensor 19. The control unit 11 activates the second sensor 20 and detects the second sensor information with a medium physical privacy disclosure degree.

The subject whose health state is determined to be abnormal at the first stage stays in the living space where the information processing device 10 is installed. The control unit 11 outputs a motion instruction indicating that the second sensor 20 can image the face of the subject, to the drive mechanism 18. The second sensor 20 images the face of the subject and detects and tracks the face image.

The control unit 11 stores the detected face image of the subject in the auxiliary storage unit 14. The control unit 11 may store a feature amount of the face image of the subject in the auxiliary storage unit 14, in addition to the face image of the subject.

The control unit 11 determines whether or not there is an abnormality in the health of the subject on the basis of the second sensor information. In a case of determining that there is an abnormality in the health of the subject, the control unit 11 executes the test program as the third stage because more detailed health observation is needed.

The control unit 11 switches the detection means from the second sensor 20 to the third sensor 21. When the detection means is switched, the control unit 11 may cause the display unit 15 to display the switching of the sensor. This is to consider the privacy of the subject.

In a case of determining that there is no abnormality in the health of the subject, the control unit 11 returns to the acquisition of the biological information by the first sensor 19. The first sensor 19 activated at this time is a different type of a first sensor 19 from the first sensor 19 activated first. The control unit 11 determines the health abnormality of the subject again, according to first sensor information acquired by the new first sensor 19.

The control unit 11 compares the number of first sensors 19 activated so far and the total number of first sensors 19 included in the information processing device 10. In a case where "the number of first sensors 19 activated so far" is less than "the total number of first sensors 19 included in the information processing device 10", the control unit 11 continues to acquire the biological information by the first sensor 19.

Note that, when acquiring the biological information, in addition to the means for switching the different types of first sensors 19, means for sequentially acquiring different portions including the arms, the torso, the legs, or the like by the same first sensor 19 may be used.

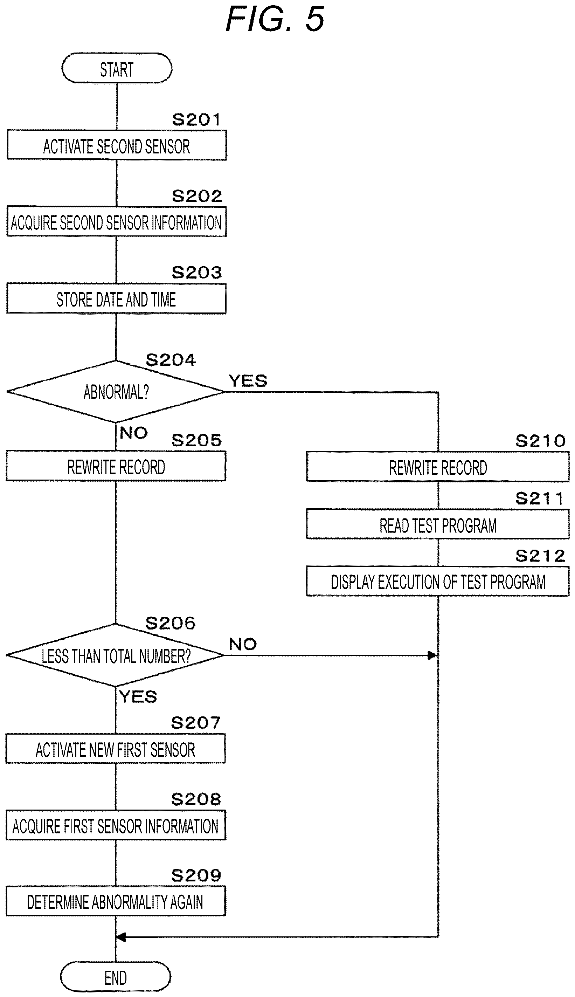

Fig. 5 is a flowchart illustrating the processing procedure of the program 140 executed by the information processing device 10. The control unit 11 of the information processing device 10 executes the following processing on the basis of the program 140.

The control unit 11 activates the second sensor 20 (step S201). The second sensor 20 detects and tracks the face image of the subject. The second sensor 20 detects the second sensor information of the subject. The control unit 11 acquires the second sensor information detected by the second sensor 20 (step S202). The control unit 11 stores a date and time when the second sensor information is detected in the health state DB 150 (step S203).

The control unit 11 determines an abnormality in the health state according to the method described above, on the basis of the acquired second sensor information (step S204). In a case of determining that there is no abnormality in the health state (step S204: NO), the control unit 11 rewrites a record in the health state field to "no abnormality" (step S205). The control unit 11 determines whether or not "the number of first sensors 19 activated so far" is less than "the total number of first sensors 19 included in the information processing device 10" (step S206).

In a case where number information stored in the auxiliary storage unit 14 is less than the total number of first sensors 19 (step S206: YES), the control unit 11 activates the different type of the first sensor 19 from the first sensor 19 activated first (step S207). The new first sensor 19 detects the first sensor information of the subject. The control unit 11 acquires the first sensor information detected by the new first sensor 19 (step S208). The control unit 11 determines an abnormality in the health state again on the basis of the acquired first sensor information (step S209).

On the other hand, in a case where the number information stored in the auxiliary storage unit 14 is not less than the total number of first sensors 19 (step S206: NO), the control unit 11 ends the series of processing.

In a case of determining that there is an abnormality in the health state (step S204: YES), the control unit 11 rewrites the record in the health state field to "abnormal" (step S210). The control unit 11 reads the test program stored in the auxiliary storage unit 14 (step S211). The control unit 11 makes the display unit 15 display the execution of the test program (step S212).

### [Health abnormality determination at third stage]

At the third stage, an individual and specific diagnosis is performed on the subject determined to have an abnormality in the second stage. The individual and specific diagnosis is performed on the basis of the test program stored in the auxiliary storage unit 14.

The control unit 11 activates the third sensor 21 that detects biological information with a higher physical privacy disclosure degree than the first sensor 19 and the second sensor 20. The third sensor 21 is, for example, a camera, a microphone, or the like. The control unit 11 acquires the third sensor information from the third sensor 21, according to a type of the test program.

In the present embodiment, description will be made as assuming that the control unit 11 executes a test program regarding the stroke. However, the present embodiment is not limited to this, and the test program may be the stress check, the walking analysis, or the like.

When executing the test program regarding the stroke, the control unit 11 activates the camera and the microphone as the third sensors 21. The control unit 11 makes the display unit 15 display instruction content or outputs a voice instruction by the speaker 17. The subject performs a predetermined motion as instructed. The control unit 11 acquires the third sensor information from the camera and the microphone. The third sensor information includes whether or not the face is distorted, whether or not the upper limb can be elevated, and presence or absence of the dysarthria. A detailed determination method of the health abnormality based on the third sensor information will be described later in the fifth embodiment.

Note that the control unit 11 may output a conceivable disease name, a nearby hospital, or the like to the display unit 15, according to the result of the test program. Furthermore, the control unit 11 may notify an information terminal owned by the subject or an information terminal owned by another user (family member, medical worker, or the like) related to the subject, of the result of the abnormality determination of the health state or the result of the test program, via the communication unit 13.

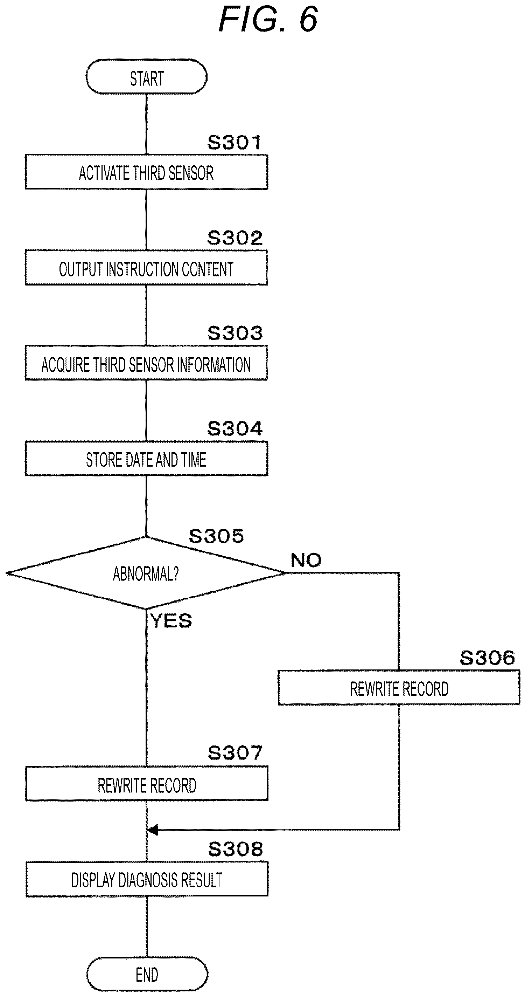

Fig. 6 is a flowchart illustrating a processing procedure of the program 140 executed by the information processing device 10. The control unit 11 of the information processing device 10 executes the following processing on the basis of the program 140.

The control unit 11 activates the third sensor 21 (step S301). The control unit 11 outputs instruction content to the display unit 15 or the speaker 17 (step S302). The third sensor 21 detects the third sensor information of the subject. The control unit 11 acquires the third sensor information detected by the third sensor 21 (step S303). The control unit 11 stores a date and time when the third sensor information is detected in the health state DB 150 (step S304).

The control unit 11 determines an abnormality in the health state according to a method to be described later in the fifth embodiment, on the basis of the acquired third sensor information (step S305). In a case of determining that there is no abnormality in the health state (step S305: NO), the control unit 11 rewrites a record in the health state field to "no abnormality" (step S306).

On the other hand, in a case of determining that there is an abnormality in the health state (step S305: YES), the control unit 11 rewrites the record in the health state field to "abnormal" (step S307). The control unit 11 makes the display unit 15 display the diagnosis result (step S308) .

As described above, according to the first embodiment, the information processing device 10 can detect the pieces of biological information having the different privacy disclosure degrees, by switching the sensors in a stepwise manner. Furthermore, the sensor such as the LiDAR, the camera, the microphone, the event drive imaging device, the millimeter wave sensor, the ultrasonic sensor, or the thermographic camera can detect the biological information in a non-contact manner. This makes it possible to monitor whether or not the subject has an abnormality without disturbing a daily life of the subject. Moreover, since it is not necessary for the subject to voluntarily activate the program for performing the individual and specific diagnosis, convenience is improved.

### [Second Embodiment]

In a second embodiment, a mode will be described in which abnormality determination based on second sensor information is skipped and a test program is executed. Note that content same as that in the first embodiment is denoted with the same reference numeral, and description thereof is omitted.

An information processing device 10 skips a second stage in a case where first sensor information includes "characteristic biological information indicating a specific symptom" (hereinafter, referred to as characteristic biological information) and in a case where an abnormality level of the first sensor information is high.

First, a case where the first sensor information includes the characteristic biological information will be described.

A control unit 11 stores the characteristic biological information in an auxiliary storage unit 14 in advance. The characteristic biological information includes, for example, falling, facial palsy, or an utterance abnormality. When the falling, the facial palsy, or the utterance abnormality occurs in the subject, the stroke is suspected. Therefore, by skipping the second stage and executing the test program at the third stage early, this can be led to early detection of diseases.

The control unit 11 compares the characteristic biological information with the first sensor information acquired from a first sensor 19, when determining a health abnormality. In a case of determining that the characteristic biological information is included in the first sensor information, the control unit 11 skips the second stage and executes the test program. The test program to be executed is a test program corresponding to a disease that is suspected based on the first sensor information. For example, in a case where the first sensor information includes the falling, the facial palsy, or the utterance abnormality, a test program for diagnosing the stroke is executed.

Next, a case will be described where the abnormality level of the first sensor information is high.

The control unit 11 stores a predetermined value according to the health abnormality in the auxiliary storage unit 14 in advance. Quantitative first sensor information used to determine the health abnormality includes, for example, a blood pressure, a frequency of a body shock or thrill, and asymmetry of the facial palsy.

In a case where the abnormality level of the first sensor information is high, the control unit 11 skips the activation of a second sensor 20 and executes the test program. The test program to be executed is a test program corresponding to a disease that is suspected based on the first sensor information.

In a case where an abnormality of the blood pressure of a subject is determined is exemplified, the auxiliary storage unit 14 stores a numerical value regarding the blood pressure according to a level of the abnormality level. Specifically, the auxiliary storage unit 14 stores the maximum blood pressure: lower than 135 mmHg and the minimum blood pressure: lower than 85 mmHg, as normal values of the blood pressure. The auxiliary storage unit 14 stores the maximum blood pressure: lower than 175 mmHg and the minimum blood pressure: lower than 105 mmHg, as blood pressures with a low abnormality level. The auxiliary storage unit 14 stores the maximum blood pressure: equal to or higher than 175 mmHg and the minimum blood pressure: equal to or higher than 105 mmHg, as blood pressures with a high abnormality level.

The control unit 11 determines the abnormality level according to the maximum blood pressure and the minimum blood pressure of the subject.

For example, the control unit 11 acquires numerical values of the maximum blood pressure: 130 mmHg and the minimum blood pressure: 80 mmHg from the first sensor 19. The control unit 11 determines that the subject has no abnormality, on the basis of the predetermined value stored in the auxiliary storage unit 14. The control unit 11 activates the different type of the first sensor 19 from the first sensor 19 activated first and continues to acquire the biological information.

For example, the control unit 11 acquires numerical values of the maximum blood pressure: 160 mmHg and the minimum blood pressure: 90 mmHg from the first sensor 19. The control unit 11 determines that the subject has the blood pressure with the low abnormality level, on the basis of the predetermined value stored in the auxiliary storage unit 14. The control unit 11 proceeds to the second stage and activates the second sensor 20.

For example, the control unit 11 acquires numerical values of the maximum blood pressure: 190 mmHg and the minimum blood pressure: 110 mmHg from the first sensor 19. The control unit 11 determines that the subject has the blood pressure with the high abnormality level, on the basis of the predetermined value stored in the auxiliary storage unit 14. The control unit 11 skips the second stage and executes the test program. The test program to be executed is a test program for diagnosing a diseases that is likely to be caused by the high blood pressure.

Note that, in a case where the abnormality level of the asymmetry of the facial palsy is high, a test program regarding the stroke is executed. In a case where the abnormality level of the body shock or thrill is high, a test program regarding the Parkinson's disease is executed.

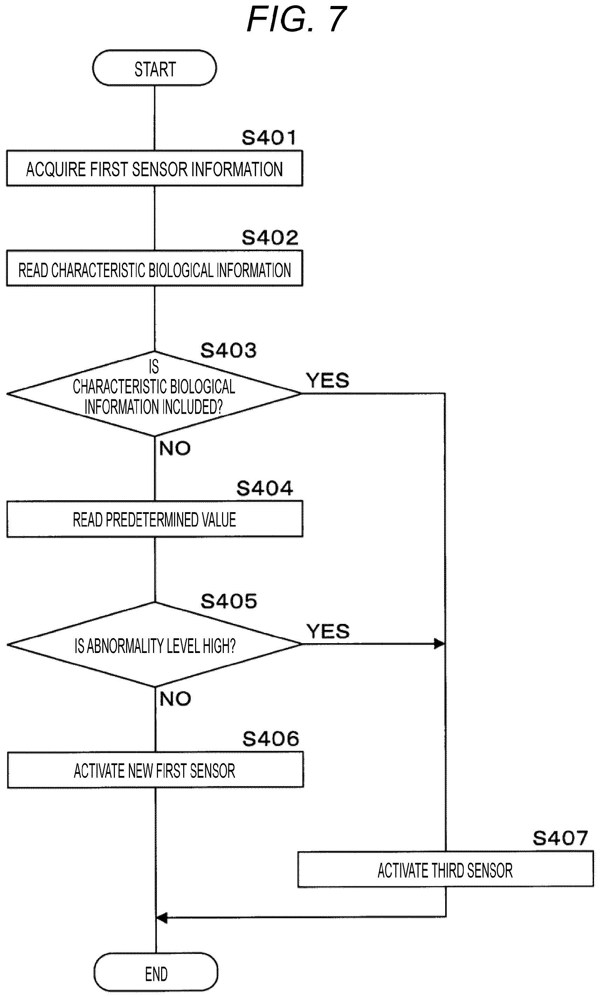

Fig. 7 is a flowchart illustrating a processing procedure of a program 140 executed by the information processing device 10. The control unit 11 of the information processing device 10 executes the following processing on the basis of the program 140.

The control unit 11 acquires the first sensor information (step S401). The control unit 11 reads the characteristic biological information stored in the auxiliary storage unit 14 (step S402). After comparing the acquired first sensor information with the read characteristic biological information, the control unit 11 determines whether or not the first sensor information of the subject includes the characteristic biological information (step S403).

In a case of determining that the characteristic biological information is not included (step S403: NO), the control unit 11 reads "the predetermined value according to the health abnormality" stored in the auxiliary storage unit 14 (step S404). On the other hand, in a case of determining that the characteristic biological information is included (step S403: YES), the control unit 11 activates a third sensor 21 (step S407).

After comparing the predetermined value included in the acquired first sensor information with the read "predetermined value according to the health abnormality", the control unit 11 determines whether or not the abnormality level is high (step S405). Note that, in a case where the abnormality level output from the machine learning model described in the first embodiment is equal to or higher than a predetermined level, the procedure may proceed to step S407.

In a case of determining that the abnormality level is not high (step S405: NO), the control unit 11 activates the different type of the first sensor 19 from the first sensor 19 activated first (step S406). On the other hand, in a case of determining that the abnormality level is high (step S405: YES), the control unit 11 activates the third sensor 21 (step S407).

Note that, in a case where it is determined that the abnormality level is low, the procedure may proceed to the second stage and the acquisition of the biological information may be continued.

As described above, according to the second embodiment, the test program can be executed while skipping the abnormality determination based on the second sensor information, and it is possible to quickly detect the disease with high treatment urgency such as brain infarction.

### [Third Embodiment]

In a third embodiment, a mode will be described in which a microphone is activated as a first sensor 19 and a LiDAR is activated as a second sensor 20 so as to determine a health state. Note that content same as that in the first embodiment is denoted with the same reference numeral, and description thereof is omitted.

An information processing device 10 activates a microphone as the first sensor 19. The information processing device 10 activates the LiDAR as the second sensor 20. The information processing device 10 accurately determines a health abnormality, by acquiring biological information by combining a plurality of sensors.

The microphone detects voice information of a subject. A control unit 11 acquires the voice information of the subject from the microphone. The control unit 11 determines an utterance abnormality by a method such as staging using the utterance clarity described above as an index. Note that the control unit 11 may determine the utterance abnormality using an utterance (for example, daily uttered voice) of the subject, instead of an utterance test.

In a case of determining that there is no utterance abnormality, the control unit 11 activates the first sensor 19 different from the microphone. On the other hand, in a case of determining that the subject has the utterance abnormality, the control unit 11 activates the LiDAR. The biological information acquired by a combination of the microphone and the LiDAR can improve accuracy of abnormality determination of the health state.

The control unit 11 acquires a face image of the subject from the LiDAR. After comparing the face image acquired from the LiDAR with a face image at a healthy time stored in an auxiliary storage unit 14, the control unit 11 determines facial palsy. Specifically, the control unit 11 determines the facial palsy, on the basis of whether or not the face of the subject is asymmetric. If the face is asymmetric, this means "facial palsy occurs", and if the face is not asymmetric, this means "no facial palsy".

More specifically, the face image acquired from the LiDAR is compared with the face image at the healthy time stored in the auxiliary storage unit 14. If feature points (forehead, eyebrows, eyes, cheeks, corners of the mouth, or the like) in a half of the face of the subject are lowered, it is determined that "the facial palsy occurs", and if the feature points (forehead, eyebrows, eyes, cheeks, corners of the mouth, or the like) in the half of the face of the subject are not lowered, it is determined that "no facial palsy occurs".

In a case of determining that the facial palsy does not occur, the control unit 11 returns to a first stage and continues to acquire the biological information. On the other hand, in a case of determining that the facial palsy occurs, the control unit 11 proceeds to a third stage. This is because a risk of the stroke is high when both of the utterance abnormality and the facial palsy occur.

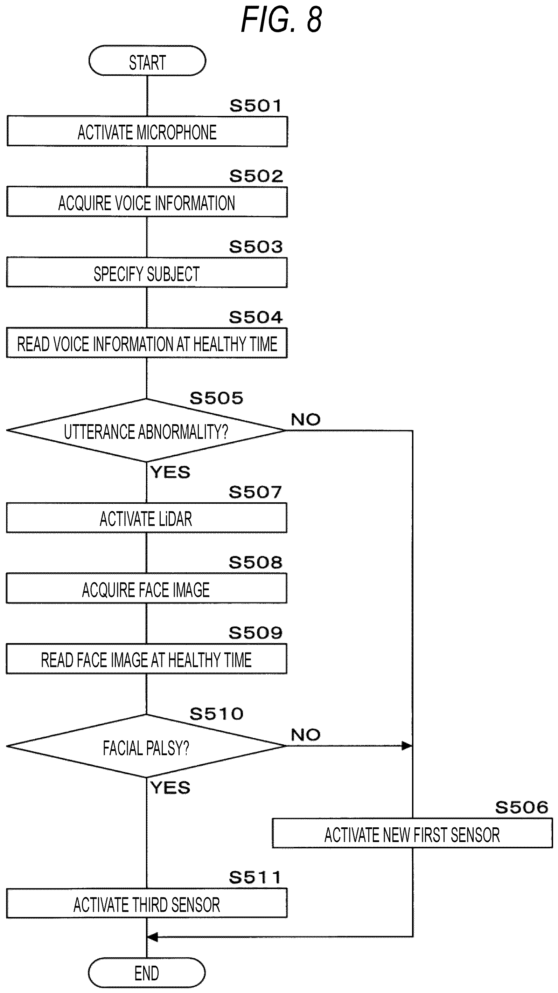

Fig. 8 is a flowchart illustrating a processing procedure of a program 140 executed by the information processing device 10. The control unit 11 of the information processing device 10 executes the following processing on the basis of the program 140.

The control unit 11 activates the microphone as the first sensor 19 (step S501). The microphone detects the voice information of the subject. The control unit 11 acquires the voice information from the microphone (step S502). The control unit 11 specifies the subject on the basis of the acquired voice information (step S503).

The control unit 11 reads the voice information at the healthy time stored in the auxiliary storage unit 14 (step S504). The control unit 11 compares the voice information acquired from the microphone with the read voice information and determines whether or not the utterance abnormality occurs (step S505).

In a case of determining that no utterance abnormality occurs (step S505: NO), the control unit 11 activates a different type of the first sensor 19 from the microphone (step S506). On the other hand, in a case of determining that the utterance abnormality occurs (step S505: YES), the control unit 11 activates the LiDAR (step S507).

The LiDAR detects the face image of the subject. The control unit 11 acquires the face image from the LiDAR (step S508). The control unit 11 reads the face image at the healthy time stored in the auxiliary storage unit 14 (step S509). The control unit 11 compares the face image acquired from the LiDAR with the read face image and determines whether or not the facial palsy occurs (step S510). Note that, whether or not the facial palsy occurs may be determined from asymmetry property of the face image acquired from the LiDAR.

In a case of determining that no facial palsy occurs (step S510: NO), the control unit 11 activates a different type of the first sensor 19 from the LiDAR (step S506). On the other hand, in a case of determining that the facial palsy occurs (step S510: YES), the control unit 11 activates the third sensor 21 (step S511).

As described above, according to the third embodiment, by acquiring the biological information by combining the plurality of sensors, it is possible to accurately determine the health abnormality.

### [Fourth Embodiment]

In a fourth embodiment, a mode will be described in which a LiDAR is activated as a first sensor 19 and a camera is activated as a second sensor 20 so as to determine a health state. By adding biological information acquired by the camera to biological information acquired by the LiDAR, for example, falling state determination accuracy is improved. Note that content same as that in the first embodiment is denoted with the same reference numeral, and description thereof is omitted.

An information processing device 10 activates the LiDAR as the first sensor 19. The information processing device 10 activates the camera as the second sensor 20. The information processing device 10 integrates respective pieces of information acquired by the LiDAR and the camera and acquires integrated information. Specifically, the information processing device 10 integrates an image acquired by the LiDAR with an image acquired by the camera. The information processing device 10 determines whether or not a subject falls on the basis of the integrated image.

When integrating the images, a control unit 11 masks a specific region in the image imaged by the camera. The control unit 11 specifies a portion to be masked on the basis of the image acquired from the LiDAR. The portion to be masked is, for example, a region of a face portion where an individual is easily identified. This is to consider privacy.

The control unit 11 specifies a posture of the subject from the masked image (hereinafter, referred to as masking image). The posture of the subject includes standing, sitting, falling, or the like. The image of the subject used for specification is, for example, an image in which a face portion of an entire body is acquired by the LiDAR, that is, an image from the neck to the bottom imaged by the camera.

The control unit 11 specifies a face portion with respect to an entire body image acquired by the camera, by a face recognition algorithm using a machine learning model such as Openpose, region based convolutional neural networks (R-CNN), or you only look once (YOLO). The control unit 11 applies the image of the face portion acquired from the LiDAR only to the specified face portion and performs masking. Since the LiDAR image and the camera image are integrated in the masking image, it is possible to achieve both of considering privacy and an abnormality determination detection function.

Note that the falling of the subject may be determined using a machine learning model such as an SVM, a transformer, or an LSTM. In this case, the control unit 11 determines the falling of the subject by inputting the masking image of the subject into a model that has learned to classify the integrated image into any one of "standing", "sitting", or "falling" when the integrated image of the camera and the LiDAR is input.

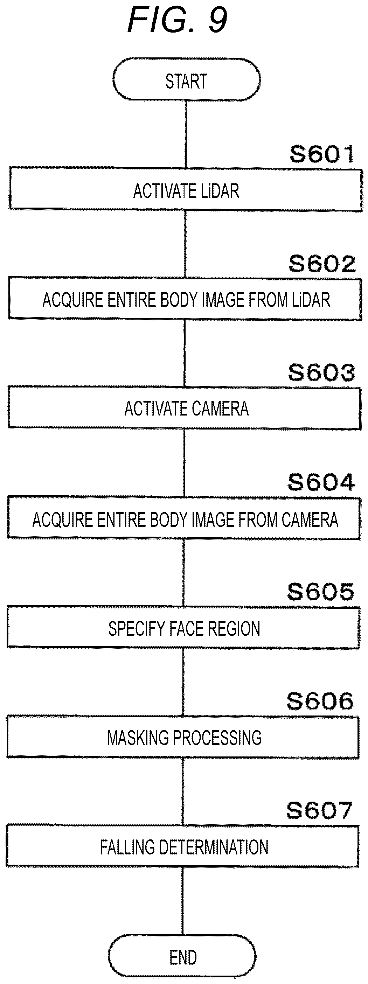

Fig. 9 is a flowchart illustrating a processing procedure of a program 140 executed by the information processing device 10. The control unit 11 of the information processing device 10 executes the following processing on the basis of the program 140.

The control unit 11 activates the LiDAR as a first stage (step S601). The control unit 11 acquires the entire body image of the subject from the LiDAR (step S602). The control unit 11 activates the camera as a second stage (step S603). The control unit 11 acquires the entire body image of the subject from the camera (step S604).

The control unit 11 specifies a face region by the face recognition algorithm, from the entire body image acquired by the camera (step S605). The control unit 11 applies the face image acquired by the LiDAR to the specified face region and performs masking (step S606). The control unit 11 determines whether or not the subject has fallen on the basis of the masking image (step S607).

As described above, according to the fourth embodiment, by using the masking image for the falling determination, it is possible to realize both of considering the privacy and the abnormality determination detection function. Furthermore, by activating the LiDAR as the first sensor 19, it is possible to acquire the biological information in a state where a privacy disclosure degree is low.

### [Fifth Embodiment]

In a fifth embodiment, a mode will be described in which a test program regarding a stroke is executed as a diagnosis for determining whether or not a brain dysfunction occurs. Hereinafter, a case where the test program is a CPSS used for a stroke diagnosis will be described. However, the brain dysfunction to be determined is not particularly limited, and for example, dementia, aphasia, a temporary recognition function decline caused by a brain infarction, and the like are exemplified, in addition to the stroke.

The CPSS is a diagnosis for capturing a facial distortion, a state of upper limb elevation, and a sign of dysarthria and checking a possibility that the stroke occurs.

In order to acquire a face image, an arm image, and voice information of a subject to be necessary for the CPSS, an information processing device 10 activates a third sensor 21. In this case, the third sensor 21 is a combination of a camera and a microphone. The third sensor 21 acquires biological information of the subject at an appropriate timing, under control by the control unit 11.

The control unit 11 causes a speaker 17 to issue a predetermined voice instruction. The subject who takes the CPSS provides biological information regarding the face distortion, the state of the upper limb elevation, and the dysarthria to the information processing device 10, in accordance with the voice instruction of the speaker 17. Note that, in addition to the voice instruction by the speaker 17, instruction content of the test program may be displayed on a display unit 15.

Hereinafter, details of capturing the face distortion, the state of the upper limb elevation, and the sign of the dysarthria will be described.

### [Face distortion]

The speaker 17 issues a voice instruction such as "smile and show your teeth" to the subject. The control unit 11 acquires a smiling face image of the subject by the camera. When the smiling face of the subject is bilaterally symmetrical, the control unit 11 determines that the subject is normal, and when the face is not bilaterally symmetrical, the control unit 11 determines that the subject is abnormal. Specifically, the control unit 11 calculates a left and right cosine similarity or a correlation and specifies symmetry.

Note that the facial distortion determination may be performed using a machine learning model such as a CNN or an SVM. In this case, the control unit 11 determines the face distortion of the subject by inputting the face image of the subject into a model that has learned to classify the face image into "distorted" or "not distorted" when the face image is input.

### [Upper limb elevation]

The speaker 17 issues a voice instruction such as "close your eyes and keep your arms raised for 10 seconds" to the subject. The control unit 11 acquires an arm image of the subject by the camera. The control unit 11 specifies a joint point of the subject, and in a case where the joint point is located at a position higher than a predetermined position, the control unit 11 determines as "elevated", and in a case where the joint point is located at a position lower than the predetermined position, the control unit 11 determines as "not elevated".

If both arms of the subject are raised or not raised, the control unit 11 determines that the subject is normal, and if only one arm is not raised or raising degrees of both arms are not the same, or the raised arm cannot be held, the control unit 11 determines that the subject is abnormal.

Note that the upper limb elevation determination may be performed using the machine learning model such as the CNN or the SVM. In this case, the control unit 11 determines the upper limb elevation of the subject by inputting the arm image of the subject into a model that has learned to classify the arm image into "elevated" or "not elevated" when the arm image is input.

### [Dysarthria]

The speaker 17 outputs a predetermined voice to the subject. The output voice is a simple question sentence that prompts the subject to utter such as "where did you go yesterday?" or "what is the weather today?". The control unit 11 acquires voice information regarding utterance content by the microphone. The control unit 11 determines that the voice information of the subject is normal if the subject accurately speaks without delay and determines that the voice information is abnormal if the subject speaks unclear words or wrong words or utters no word.

Note that the determination on the dysarthria may be performed using a machine learning model such as a neural network, an SVM, a transformer, or an LSTM. In this case, the control unit 11 determines the dysarthria of the subject by inputting the utterance content of the subject into a model that has learned to classify utterance data into "dysarthria" or "no dysarthria" when the utterance data is input.

Finally, the control unit 11 acquires CPSS information including an item for which an abnormality is determined and the number of items, regarding the face distortion, the state of the upper limb elevation, and the dysarthria. The control unit 11 outputs a probability or a score at which the stroke occurs, on the basis of the acquired CPSS information.

When the probability is output, an average value of probabilities regarding an abnormality level output by the machine learning model in each diagnosis is output. For example, in a case where the machine learning model outputs a face distortion test: 60%, an upper limb elevation test: 60%, and a dysarthria test: 90%, the probability of the stroke is output as 70%. The output probability may be displayed on the display unit 15.

When the score is output, a total score of each diagnosis set as "one point" for "abnormal" and "zero point" for "no abnormality" is output. For example, if the face distortion: one point, the upper limb elevation: one point, and the dysarthria: zero point, the stroke score is output as two points. The output score may be displayed on the display unit 15.

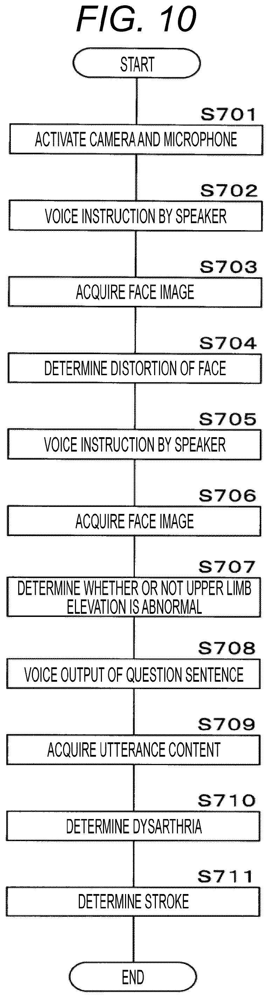

Fig. 10 is a flowchart illustrating a processing procedure of a program 140 executed by the information processing device 10. The control unit 11 of the information processing device 10 executes the following processing on the basis of the program 140.

The control unit 11 activates the camera and the microphone as the third sensors 21 (step S701). The control unit 11 causes the speaker 17 to issue the voice instruction to acquire the smiling face image of the subject (step S702). The camera images the face image of the subject. The control unit 11 acquires the face image of the subject from the camera (step S703). The control unit 11 determines whether or not the face is distorted on the basis of the face image of the subject (step S704) .

The control unit 11 causes the speaker 17 to issue a voice instruction to acquire the biological information of the upper limb elevation (step S705). The camera images a state where the subject is raising the upper limb. The control unit 11 acquires the arm image of the subject (step S706). The control unit 11 determines whether or not the upper limb elevation is abnormal on the basis of the arm image of the subject (step S707) .

The control unit 11 causes the speaker 17 to output a predetermined question sentence (step S708). The microphone acquires the voice information including the utterance content of the subject (step S709). The control unit 11 determines whether or not the dysarthria occurs on the basis of the utterance content of the subject (step S710) .

The control unit 11 outputs the probability or the score at which the stroke occurs, according to the abnormality determination specified in steps S704, S707, and S710 (step S711).

As described above, according to the fifth embodiment, by executing the test program for determining whether or not the brain dysfunction occurs, it is possible to perform abnormality determination. Furthermore, since it is not necessary for the subject to voluntarily activate the test program, convenience is improved. Moreover, since the test program can be executed without intervention of a third party, the subject alone can notice an abnormality in the own health state.

### [Modifications]

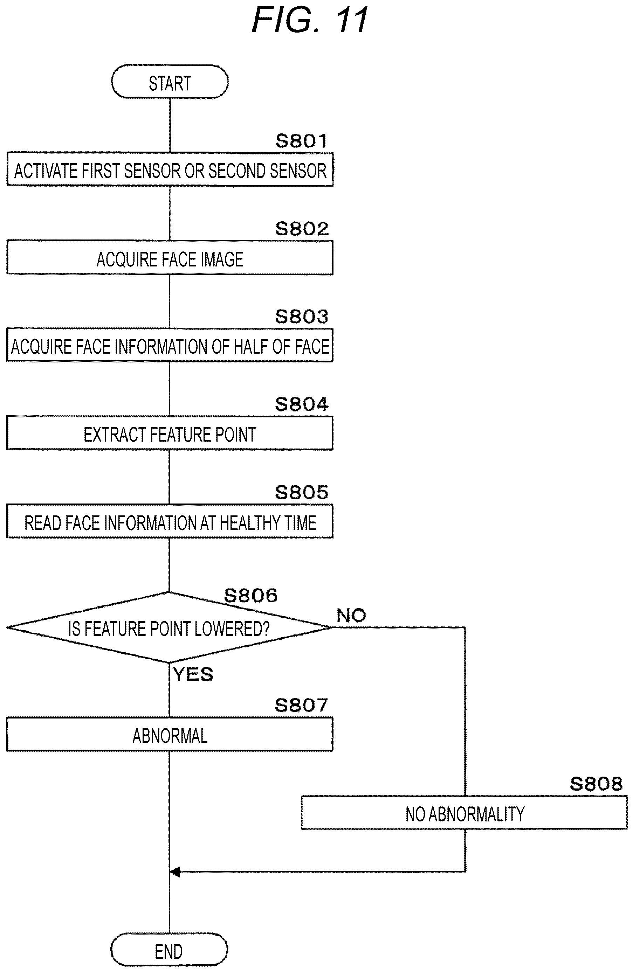

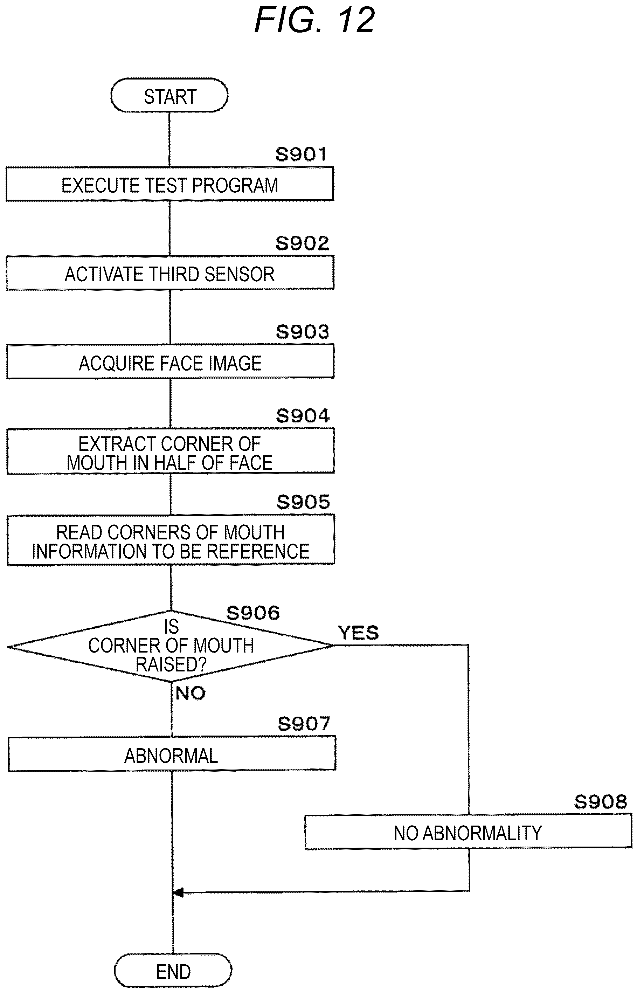

In a mode in which the first sensor 19, the second sensor 20, or the third sensor 21 images the face of the subject, processing when an abnormality is determined on the basis of the feature point (for example, corners of the mouth) of the face is illustrated in Figs. 11 and 12.

Fig. 11 illustrates a case where the first sensor 19 or the second sensor 20 for imaging the face of the subject is activated to perform the abnormality determination.

The control unit 11 acquires the face image from the first sensor 19 or the second sensor 20 and acquires the face information of the half of the face from the face image. The control unit 11 extracts the feature points on the left side and the feature points on the right side included in the face image and compares positions of the feature points on the left and right with the face information at the healthy time.

In a case where the position of one of the right and left feature points is lowered as compared with the face information at the healthy time, the control unit 11 determines that the subject is abnormal. Furthermore, in a case where the positions of both feature points are lowered as compared with the face information at the healthy time, the control unit 11 may determine that the subject is abnormal. Alternatively, in a case where the position of the feature point on one side is lower than the position of the feature point on the opposite side, by comparing the feature points on the left and right with each other, the control unit 11 determines that the subject is abnormal.

Fig. 11 is a flowchart illustrating a processing procedure of the program 140 executed by the information processing device 10. The control unit 11 of the information processing device 10 executes the following processing on the basis of the program 140.

The control unit 11 activates the first sensor 19 or the second sensor 20 (step S801). The first sensor 19 or the second sensor 20 images the face of the subject. The control unit 11 acquires the face image of the subject from the first sensor 19 or the second sensor 20 (step S802). The control unit 11 acquires the face information of the half of the face from the face image (step S803). The control unit 11 extracts the feature point included in the face information of the half of the face (step S804).

The control unit 11 reads the face information at the healthy time stored in the auxiliary storage unit 14 (step S805). The control unit 11 determines whether or not the extracted feature point is lowered, on the basis of the face information at the healthy time and the extracted feature point (step S806).

In a case where the extracted feature point is lower than the face information at the healthy time (step S806: YES), the control unit 11 determines that the subject is abnormal (step S807). On the other hand, in a case where the extracted feature point is not lower than the face information at the healthy time (step S806: NO), the control unit 11 determines that the subject is not abnormal (step S808).

Note that the control unit 11 may perform abnormality determination by comparing the left and right feature points with each other, in the processing in step S806. In this case, the processing in steps S803 and S805 is not essential.

Fig. 12 illustrates a case where the third sensor 21 that images the face of the subject is activated to perform the abnormality determination after a test program for instructing the subject to make a smiling face is executed.

The control unit 11 acquires a face image (for example, face image in which subject is smiling) from the third sensor 21 and acquires the corner of the mouth in the half of the face from the face image. The control unit 11 extracts the corners of the mouth on the left side and the right side included in the face image, and compares positions of the left and right corners of the mouth with reference corners of the mouth.

In a case where the position of one of the right and left corners of the mouth is lowered as compared with the face information at the healthy time, the control unit 11 determines that the subject is abnormal. Furthermore, in a case where both of the corners of the mouth are not raised, as compared with the reference corners of the mouth, the control unit 11 determines that the subject is abnormal. Alternatively, in a case where the left and right corners of the mouth are compared and the position of the corner of the mouth on one side is not higher than the position of the corner of the mouth on the opposite side, the control unit 11 determines that the subject is abnormal.

Fig. 12 is a flowchart illustrating a processing procedure of the program 140 executed by the information processing device 10. The control unit 11 of the information processing device 10 executes the following processing on the basis of the program 140.

The control unit 11 executes the test program (step S901). The control unit 11 activates the third sensor 21 (step S902). The third sensor 21 acquires the face image of the subject. The control unit 11 acquires the face image of the subject (step S903). The control unit 11 extracts the corner of the mouth in the half of the face from the face image (step S904).

The control unit 11 reads corners of the mouth information, to be the reference, stored in the auxiliary storage unit 14 (step S905). The control unit 11 determines whether or not the corner of the mouth in the half of the face extracted from the face image is raised, on the basis of the read corners of the mouth information to be the reference and the corner of the mouth in the half of the face extracted from the face image (step S906).

In a case where the extracted corner of the mouth is not raised than the corners of the mouth information to be the reference (step S906: NO), the control unit 11 determines that the subject is abnormal (step S907). On the other hand, in a case where the extracted corners of the mouth are raised as high as the corners of the mouth information to be the reference (step S906: YES), the control unit 11 determines that the subject is not abnormal (step S908).

Note that the control unit 11 may perform the abnormality determination by comparing the left and right corners of the mouth with each other, in the processing in step S906. In this case, the processing in steps S904 and S905 is not essential, and both of the left and right corners of the mouth are extracted before the left and right corners of the mouth are compared.

According to the modification described above, it is possible to determine the abnormality of the subject on the basis of the specific portion of the face. Note that, in the modification described above, a case has been described where the abnormality is determined on the basis of the feature points (for example, corners of the mouth) of the face. However, the abnormality may be determined on the basis of the feature amount extracted from the shock, the thrill, the faintness, or the utterance.

### [Sixth Embodiment]

Note that content same as that in the first embodiment is denoted with the same reference numeral, and description thereof is omitted. In a sixth embodiment, processing is executed so as to lower privacy disclosure of a subject as possible when determination is made by appropriately using a first sensor 19, a second sensor 20, and a third sensor 21 and a determination result is output.

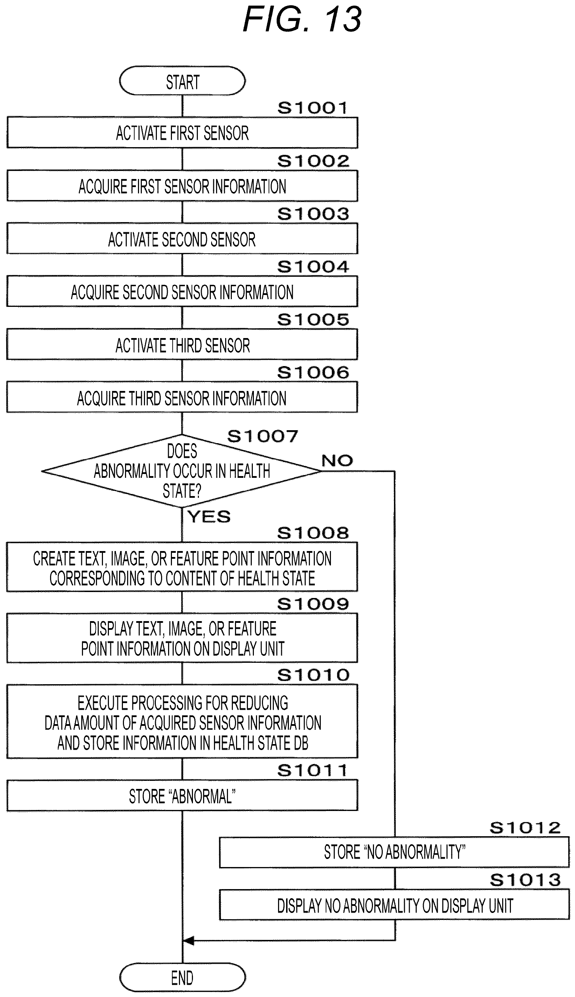

Fig. 13 is a flowchart illustrating an example of a processing procedure of a program 140 executed by an information processing device 10 according to the sixth embodiment. A control unit 11 of the information processing device 10 executes the following processing on the basis of the program 140.

The control unit 11 activates the first sensor 19 (step S1001). The first sensor 19 detects first sensor information of the subject. The control unit 11 acquires the first sensor information detected by the first sensor 19 together with a date and time when the first sensor information is detected (step S1002). The control unit 11 may stop the first sensor 19.

The control unit 11 activates the second sensor 20 (step S1003). The second sensor 20 detects second sensor information of the subject. The control unit 11 acquires the second sensor information detected by the second sensor 20 together with a date and time when the second sensor information is detected (step S1004). The control unit 11 may stop the second sensor 20.

The control unit 11 activates the third sensor 21 (step S1005). The third sensor 21 detects third sensor information of the subject. The control unit 11 acquires the third sensor information detected by the third sensor 21 together with a date and time when the third sensor information is detected (step S1006). The control unit 11 may stop the third sensor 21.

The second sensor 20 and the third sensor 21 detect biological information with a higher physical privacy disclosure degree than the first sensor 19. Therefore, the control unit 11 may activate only a sensor that is determined to be related to the subject, among the second sensors 20, on the basis of the first sensor information. Similarly, the control unit 11 may activate the sensor determined to be related to the subject among the third sensors 21, according to both or one of the first sensor information and the second sensor information and may execute a test program.

The control unit 11 determines whether or not an abnormality occurs in a health state of the subject using the first sensor information, the second sensor information, and the third sensor information (step S1007). Step S1007 is an example of first determination processing or second determination processing.

In a case where it is determined that the abnormality occurs in the health state of the subject (step S1007: YES), the control unit 11 creates a text, an image, or feature point information corresponding to content of the health state of the subject on the basis of the first sensor information, the second sensor information, and the third sensor information (step S1008). In step S1008, the control unit 11 creates data of the text, the image, the feature point information, or the like abstracted into data (meaning information belonging to first sensor information, second sensor information, and third sensor information) called so-called metadata as an example of processing for reducing a data amount. For example, from an image of a face that has come to be recognized to suspect a stroke (for example, image of facial palsy of subject), an image obtained by imaging an operation in response to instruction content in the test program (for example, image in which one of left and right corners of mouth of subject is not raised), or the like, the control unit 11 creates an ROI image trimmed within a range in which an individual cannot be identified and a distortion (for example, facial palsy) can be found or creates only a text such as "stroke of subject is suspected" or "subject has facial palsy". Furthermore, for example, from the image of the face that has come to be recognized to suspect the stroke (for example, image of facial palsy of subject), the image obtained by imaging the operation in response to the instruction content in the test program (for example, image in which one of left and right corners of mouth of subject is not raised), or the like, the control unit 11 extracts feature points (eyes, corners of eyes, mouth, corners of mouth, nose, nasalis muscle, or the like) of the face of the subject and generates feature point information such as an image including only feature points, a line connecting the feature points, position coordinates of the feature point, or angle information of the feature points. The control unit 11 displays the created text, image, or feature point information on a display unit 15 (step S1009). Step S1009 is an example of outputting metadata corresponding to content of a determination result of the first determination processing or the second determination processing.

The control unit 11 executes processing for reducing the data amount of the sensor information acquired in steps S1002, S1004, and S1006 and stores the sensor information in a health state DB 150 (step S1010). In step S1011, the control unit 11 may calculate a numerical value necessary for a diagnosis of the health state from the sensor information, and then, store the numerical value. The control unit 11 rewrites a record in a health state field of the subject into "abnormal" (step S1011) and ends the processing.

In a case where it is determined that the health state is not abnormal (step S1007: NO), the control unit 11 writes the record in the health state field of the subject as "no abnormality" and stores the record in the health state DB 150 (step S1012). The control unit 11 displays a text or an image indicating "no abnormality" on the display unit 15 (step S1013) and ends the processing.

As illustrated in Fig. 13, the control unit 11 does not need to perform a diagnosis using all of the first sensor 19, the second sensor 20, and the third sensor 21. In a case where the control unit 11 determines that the diagnosis by the second sensor 20 or the third sensor 21 is not needed, in the diagnosis based on the biological information with a lower disclosure obtained from the first sensor 19, in a stepwise manner, as described in the first embodiment to the fifth embodiment, the diagnosis by the second sensor 20 or the third sensor 21 may be omitted. In the sixth embodiment, a diagnosis result to be displayed on the display unit 15 is merely abstracted as possible, and the privacy disclosure is kept to be low without lowering health abnormality determination accuracy. Note that the first sensor 19, the second sensor 20, and the third sensor 21 may be different types of sensors or may be the same sensors having different privacy disclosure degrees.

As described above, according to the sixth embodiment, by acquiring the biological information by combining the plurality of sensors, it is possible to abstract and output (change to metadata) the determination result while improving the health abnormality determination accuracy and to avoid unnecessary privacy disclosure.

As described above, according to the first embodiment to the sixth embodiment, a program or the like that switches the method for acquiring the biological information, in a stepwise manner, according to the abnormality degree of the health state can be provided, and the health observation and the privacy ensuring can be balanced. Furthermore, since the sensor such as the LiDAR, the camera, the microphone, the event drive imaging device, the millimeter wave sensor, the ultrasonic sensor, or the thermographic camera can acquire the biological information in a non-contact manner, it is possible to monitor whether or not the subject is abnormal, without disturbing a daily life of the subject.

The embodiments disclosed herein are illustrative in all points, and are not restrictive. The technical scope of the present invention is defined on the basis of the description in claims rather than the meaning disclosed above, and includes all modifications within the meaning and scope equivalent to the claims.

### Reference Signs List

- 10: information processing device
- 10a: portable storage medium
- 11: control unit
- 12: main storage unit
- 13: communication unit
- 14: auxiliary storage unit
- 140: program (program product)
- 150: health state DB
- 15: display unit (output unit)
- 16: input unit
- 17: speaker
- 18: drive mechanism
- 19: first sensor
- 20: second sensor
- 21: third sensor

## Claims

1. A program for causing a computer to execute processing comprising:
determining an abnormality of a subject on a basis of first sensor information obtained from a first sensor; and
determining the abnormality of the subject on a basis of second sensor information obtained from a second sensor having a higher physical privacy disclosure degree than the first sensor or executing a test program regarding the abnormality on the subject, according to a determination result.

2. The program according to claim 1, wherein
in a case where it is determined that the subject is not abnormal on a basis of the second sensor information, switching to a different type of a first sensor from the first sensor is performed; and
the abnormality of the subject is determined again on a basis of first sensor information obtained from the first sensor.

3. The program according to claim 1, wherein
abnormality determination based on the second sensor information is skipped and the test program is executed, according to a degree of the abnormality of the subject determined on a basis of the first sensor information.

4. The program according to any one of claims 1 to 3, wherein
the second sensor is a second imaging device that images a face of the subject, and
the first sensor is a first imaging device that does not image the face of the subject or a first imaging device having a wider angle than the second imaging device.

5. The program according to any one of claims 1 to 4, wherein
the first sensor is a microphone, a LiDAR, or an event drive imaging device that extracts a luminance change.

6. The program according to any one of claims 1 to 5, wherein
facial palsy, a biological information abnormality, a mental abnormality, falling, shock or thrill, faintness, or an utterance abnormality is detected on a basis of the first sensor information or the second sensor information.

7. The program according to any one of claims 1 to 6, wherein
the first sensor is a microphone, the second sensor is a LiDAR,
the utterance abnormality is detected on a basis of voice information acquired by the microphone, and
the facial palsy is detected on a basis of a face image acquired by the LiDAR.

8. The program according to any one of claims 1 to 7, wherein
integrated information obtained by integrating information acquired by the LiDAR and information acquired by the camera is acquired; and
whether or not the subject has fallen is detected on a basis of the integrated information.

9. The program according to any one of claims 1 to 8, wherein
the test program determines the abnormality of the subject on a basis of third sensor information obtained from a third sensor with a higher physical privacy disclosure degree than the first sensor.

10. The program according to claim 9, for causing the computer to execute at least processing further comprising:
first determination processing for determining whether or not the subject is abnormal on a basis of biological information of the subject obtained from at least one of the first sensor, the second sensor, and the third sensor; and
processing for outputting a result of the first determination processing, and
the program for causing the computer to further execute processing comprising:
second determination processing for determining whether or not there is an abnormality regarding an item with a higher physical privacy disclosure degree than the first determination processing, on a basis of the biological information acquired from at least one of the first sensor, the second sensor, and the third sensor or biological information of the subject acquired again, according to the result of the first determination processing; and
processing for outputting a result of the second determination processing.

11. The program according to any one of claims 1 to 10, wherein
the test program is executed in a case where it is determined that the subject is abnormal on a basis of the second sensor information.

12. The program according to any one of claims 1 to 11, wherein
the test program is a diagnosis for determining whether or not a brain dysfunction occurs.

13. The program according to claim 12, wherein
a test program regarding a stroke is executed,
an image of a face or an arm of the subject imaged by the third sensor is acquired, and
the abnormality of the subject is determined on a basis of the acquired image and utterance content of the subject acquired by executing the test program.

14. The program according to any one of claims 1 to 13, wherein
a face image of the subject imaged by the first sensor or the second sensor is acquired,
face information of a half of the face is extracted from the face image, and
in a case where the face information is lower than face information to be a reference or in a case where the face information is lower than face information of an opposite half of the face, this is determined as an abnormal state.

15. The program according to any one of claims 9 to 13, wherein
the test program for instructing the subject to make a smiling face is executed,
the face image of the subject imaged by the third sensor is acquired after the execution of the test program,
a corner of the mouth in a half of the face is extracted from the face image, and
in a case where the corner of the mouth is not raised as compared with a corner of the mouth to be a reference or in a case where the corner of the mouth is not raised as compared with a corner of the mouth in the opposite half of the face, this is determined as an abnormal state.

16. A program for causing a computer to execute processing comprising:
first determination processing for determining whether or not a subject is abnormal on a basis of biological information of the subject obtained from one or a plurality of sensors; and
second determination processing for determining whether or not there is an abnormality regarding an item with a higher physical privacy disclosure degree than the first determination processing, on a basis of the biological information obtained from the one or the plurality of sensors, according to the presence or absence of the abnormality.

17. The program according to claim 16, for causing the computer to execute processing further comprising:
executing processing for reducing a data amount of a determination result when the determination result of the first determination processing or the second determination processing is output and outputting metadata corresponding to content of the determination result.

18. An information processing device comprising:
a determination unit that determines an abnormality of a subject on a basis of first sensor information obtained from a first sensor; and
a control unit that determines the abnormality of the subject on a basis of second sensor information obtained from a second sensor having a higher physical privacy disclosure degree than the first sensor or executes a test program regarding the abnormality on the subject, according to a determination result.

19. An information processing device comprising:
a first determination unit that determines whether or not a subject is abnormal on a basis of biological information of the subject obtained from one or a plurality of sensors;
a second determination unit that determines whether or not there is an abnormality regarding an item with a higher physical privacy disclosure degree than the first determination unit, on a basis of the biological information obtained from the one or the plurality of sensors; and
an output unit that outputs a determination result by the first determination unit or a determination result by the second determination unit, according to the presence or absence of the abnormality.

20. An information processing method performed by a computer, comprising:
determining an abnormality of a subject on a basis of first sensor information obtained from a first sensor; and
determining the abnormality of the subject on a basis of second sensor information obtained from a second sensor having a higher physical privacy disclosure degree than the first sensor or executing a test program regarding the abnormality on the subject, according to a determination result.

21. An information processing method performed by the computer, comprising:
first determination processing for determining whether or not a subject is abnormal on a basis of biological information of the subject obtained from one or a plurality of sensors; and
second determination processing for determining whether or not there is an abnormality regarding an item with a higher physical privacy disclosure degree than the first determination processing, on a basis of the biological information obtained from the one or the plurality of sensors, according to the presence or absence of the abnormality.
